# EUROPEAN PATENT SPECIFICATION

(11) **EP 1 176 945 B1**
(45) Date of publication and mention of the grant of the patent: **03.11.2004**
(21) Application number: 00928887.9
(22) Date of filing: 05.05.2000
(51) Int. Cl.: A61K 7/46, A61K 7/06, A61K 7/48

(54) **COSMETIC COMPOSITIONS**
KOSMETISCHE ZUSAMMENSETZUNGEN
COMPOSITIONS COSMETIQUES

(30) Priority: 07.05.1999 WO PCT/US99/09905
(43) Date of publication of application: 06.02.2002
(73) Proprietor: THE PROCTER & GAMBLE COMPANY, Cincinnati, Ohio 45202 (US)
(72) Inventor: MALTON, Peter, James, Egham Surrey TW20 0PA (GB); HOLLAND, Lynette, Anne, Makins, Hertfordshire WD2 6DM (GB); RIZZI, George, Cincinnati, OH 45224 (US); HELTOVICS, Gabor, Egham Surrey TW20 8HH (GB)
(74) Representative: Wilding, Richard Alan
(86) International application number: PCT/US2000/012418
(87) International publication number: WO 2000/067721

(56) References cited:
- WO-A-98/56341
- WO-A-98/56888
- PATENT ABSTRACTS OF JAPAN vol. 1995, no. 01, 28 February 1995 (1995-02-28) & JP 06 287127 A (SHISEIDO CO LTD), 11 October 1994 (1994-10-11)

## Description

### Field of the Invention

The present invention relates to cosmetic compositions and in particular to long-lasting fragrance compositions.

### Background to the Invention

It has long been a feature of cosmetic compositions that they comprise a fragrance. The addition of a fragrance can mask unpleasant odours or can improve consumer acceptance of a composition through delivering a pleasant smell. Indeed, the sole purpose of some compositions is the application of a pleasant odour to the skin, hair or other suitable substrate. However, for the most part, the fragrant effect of these compositions is transitory and the fragrance quickly becomes imperceptible.

Attempts have been made to improve the longevity of the fragrance. For instance, the fragrance may be formulated in such a way as to include a higher proportion of fragrance materials with a low volatility. This means the fragrance persists for longer. However, using less volatile materials restricts the fragrance characters that can be achieved. It has also been suggested that it may be possible to utilise cyclic oligosaccharides, and in particular cyclodextrins. For example, JP-A-50/63126 discloses perfume and cyclodextrin complexes for use in bath preparations and JP-A-7/241333 discloses a long-lasting, room deodorising composition containing a fragrance and cyclodextrin.

One drawback of using cyclic oligosaccharides is that they are only sparingly soluble in commonly used solvents. Attempts have been made to increase the solubility of cyclic oligosaccharides by introducing various substituents. See, for example, JP-A-6/287127, JP-A-8/176587, JP-A-10/120541, JP-A-62/161720 and JP-A-63/192706 all of which disclose compositions comprising perfume and substituted cyclodextrins. However, it is difficult to achieve a long-lasting, perceptible fragrance while at the same time having a desirable 'burst' of fragrance on application of the product. In addition, it is difficult to form a fragrance:cyclic oligosaccharide complex with the correct stability profile so that enough fragrance is released over a sufficiently long period time to provide a long lasting effect.

Surprisingly, it has been found that compositions comprising fragrance, cyclic oligosaccharide having one or more unsubstituted alkyl substituents and 50% or greater volatile solvent provide an initial 'burst' of fragrance and improved fragrance longevity.

While not wishing to be bound by theory, it is believed that the compositions of the present invention are substantially free of fragrance-cyclic oligosaccharide complexes until applied to the skin, hair or other substrate. On application, the volatile solvent evaporates providing an initial fragrance burst while the remaining fragrance complexes with the cyclic oligosaccharide and is gradually released over time. In addition, it is believed that the stability profile of the complexes formed between the fragrance and the cyclic oligosaccharides of the present invention is such that a perceptible amount of fragrance is released over a sufficiently long time to satisfy the consumers desire for long lasting fragrance.

### Summary of the invention

According to the present invention there is provided cosmetic compositions comprising:
(a) fragrance;
(b) cyclic oligosaccharide having one or more unsubstituted alkyl substituents; and
(c) 50% or greater, by weight, of volatile solvent.

The compositions of the present invention provide a long-lasting fragrance while at the same time having a 'burst' of fragrance on application.

All percentages herein are by weight of the composition unless otherwise indicated. All ratios are weight ratios unless otherwise indicated. Unless otherwise indicated, all percentages, ratios, and levels of ingredients referred to herein are based on the actual amount of the ingredient, and do not include solvents, fillers, or other materials which may be combined with the ingredient in commercially available products.

### Detailed description of the invention

The present invention relates to compositions comprising three essential elements, namely, fragrance, cyclic oligosaccharide having one or more unsubstituted alkyl substituents and 50% or greater, by weight, volatile solvent. These three elements will be described in more detail below.

### Fragrance

An essential feature of the present compositions is that they comprise fragrance material. As used herein the term "fragrance" is used to indicate any odouriferous material. Any fragrance material suitable for use in cosmetic compositions may be used herein but the fragrance will most often be liquid at ambient temperatures. Generally, the fragrance material will be present at a level of from about 0.01% to about 40%, by weight, of total composition. Preferably the fragrance material is present at a level of from about 1% to about 30%, more preferably from about 2.5% to about 25%, even more preferably from about 5% to about 20%, even more preferably still from about 10% to about 15%, by weight, of total composition.

A wide variety of chemicals are known for fragrance uses, including materials such as aldehydes, ketones and esters. More commonly, naturally occurring plant and animal oils and exudates comprising complex mixtures of various chemical components are known for use as fragrances. The fragrance materials useful herein include pro-fragrances such as acetal pro-fragrances, ketal pro-fragrances, ester pro-fragrances, hydrolyzable inorganic-organic pro-fragrances and mixtures thereof. The fragrance material may be released from the pro-fragrances in a number of ways. For example, the fragrance may be released as a result of simple hydrolysis, or by a shift in an equilibrium reaction, or by a pH-change, or by enzymatic release. The fragrances herein can be relatively simple in their compositions, comprising a single chemical, or can comprise highly sophisticated complex mixtures of natural and synthetic chemical components, all chosen to provide any desired odour.

Preferably the fragrance materials will have boiling points (BP) of about 500°C or lower, more preferably about 400°C or lower, even more preferably about 350°C or lower. The BP of many fragrance materials are given in *Perfume and Flavor Chemicals (Aroma Chemicals), Steffen Arctander (1969)*. The ClogP value of the fragrance materials useful herein is preferably greater than about 0.1, more preferably greater than about 0.5, even more preferably greater than about 1.0, even more preferably still greater than about 1.2. As used herein the term "ClogP" means the logarithm to the base 10 of the octanol/water partition coefficient. This can be readily calculated from a program called "CLOGP" which is available from Daylight Chemical Information Systems Inc., Irvine CA, USA. Octanol/water partition coefficients are described in more detail in US-A-5,578,563.

Suitable fragrance materials can be found in US-A-4,145,184, US-A-4,209,417, US-A-4,515,705, and US-A-4,152,272. Examples of fragrances useful herein include, but are not limited to, animal fragrances such as musk oil, civet, castoreum, ambergris, plant fragrances such as nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract and mixtures thereof.

Other examples of suitable fragrance materials include, but are not limited to, chemical substances such as acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-β-naphthyl ketone, methylphenylcarbinyl acetate, mugetanol, γ-nonalactone, octanal, phenyl ethyl acetate, phenyl-acetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox and mixtures thereof.

### Cyclic Oligosaccharides

A second essential element of the compositions of the present invention a cyclic oligosaccharide having one or more unsubstituted alkyl substituents. As used herein, the term "cyclic oligosaccharide" means a cyclic structure comprising six or more saccharide units. Preferred for use herein are cyclic oligosaccharides having six, seven or eight saccharide units and mixtures thereof, more preferably seven saccharide units. It is common in the art to abbreviate six, seven and eight membered cyclic oligosaccharides to α, β and γ respectively.

The compositions of the present invention preferably comprise from about 0.001% to about 40%, more preferably from about 0.1% to about 25%, even more preferably from about 1% to about 20%, especially from about 2% to about 15%, by weight, of cyclic oligosaccharide.

The cyclic oligosaccharides may comprise any suitable saccharide or mixtures of saccharides. Examples of suitable saccharides include, but are not limited to, glucose, fructose, mannose, galactose, maltose and mixtures thereof. However, preferred for use herein are cyclic oligosaccharides of glucose. Therefore, the preferred cyclic oligosaccharides for use herein are β-cyclodextrins.

The cyclic oligosaccharides for use herein must have one or more unsubstituted alkyl substituents. The alkyl substituent may be saturated or unsaturated, straight or branched chain but is preferably saturated and straight chain. Preferably, the alkyl substituent is selected from C₁-C₈ alkyl groups and mixtures thereof. More preferably the alkyl substituent is selected from C₁-C₆ alkyl groups and mixtures thereof. Even more preferably the alkyl substituent is selected from C₁-C₄ alkyl groups and mixtures thereof. Preferred alkyl substituents are ethyl and methyl, especially methyl. Therefore, the most preferred cyclic oligosaccharides for use herein are methyl-β-cyclodextrins. Preferred methyl-β-cyclodextrins are available from Wacker-Chemie GmbH, Hanns-Seidel-Platz 4, Munchen, DE under the tradename Beta W7 M1.8. As used in this paragraph, the term "and mixtures thereof' means two or more different alkyl groups are substituted on the same cyclic oligosaccharide.

The cyclic oligosaccharides are preferably substituted only by the unsubstituted alkyl substituents mentioned hereinabove. However, they may be substituted by other substituents. Examples of other suitable substituents include, but are not limited to, hydroxyalkyl groups, aryl groups, maltosyl groups, allyl groups, benzyl groups, alkanoyl groups and mixtures thereof.

Methods of modifying cyclic oligosaccharides are well known in the art. For example, see "*Methods of Selective Modifications of Cyclodextrins" Chemical Reviews (1998) Vol. 98, No.5, pp 1977-1996, Khan et al* and *US-A-5,710,268.*

The cyclic oligosaccharides preferably have an average degree of substitution of from about 0.5 to about 3.0, more preferably from about 1.0 to about 2.8, even more preferably from about 1.2 to about 2.3, especially from about 1.6 to about 1.9. As used herein the term "degree of substitution" means the average number of substituents per saccharide unit. The average number of substituents can be determined using common Nuclear Magnetic Resonance techniques known in the art.

The cyclic oligosaccharides are preferably soluble in both water and ethanol. As used herein "soluble" means at least about 0.1 g of solute dissolves in 100ml of solvent, at 25°C and 1 atm of pressure. Preferably the cyclic oligosaccharides for use herein have a solubility of at least about 1g/100ml, more preferably at least about 10g/100ml, even more preferably at least about 100g/100ml, at 25°C and 1 atm of pressure.

Preferably the weight ratio of total fragrance to cyclic oligosaccharide in the compositions of the present invention is at least about 1:1, more preferably at least about 1.5:1, even more preferably at least about 2:1.

### Volatile Solvent

A third essential element of the compositions of the present invention is that they comprise 50% or greater, by weight, of volatile solvent or mixture of volatile solvents. The solvents for use herein are preferably organic volatile solvents. Preferably, the present compositions comprise 55% or greater, more preferably 60% or greater, even more preferably 65% or greater, by weight, of volatile solvent.

As used herein, "volatile" refers to substances with a significant amount of vapour pressure under ambient conditions, as is understood by those in the art. The volatile solvents for use herein will preferably have a vapour pressure of about 2kPa or more, more preferably about 6kPa or more, at 25°C. The volatile solvents for use herein will preferably have a boiling point under one atmosphere (atm) of less than about 150°C, more preferably less than about 100°C, even more preferably less than about 90°C, even more preferably still less than about 80°C.

Preferably the volatile solvents for use herein will be relatively odourless and safe for use on human skin. Preferred volatile solvents for use herein are C₁-C₄ alcohols, volatile silicones and mixtures thereof. More preferred are C₁-C₄ alcohols and mixtures thereof. Especially preferred for use herein is ethanol.

### Optional Ingredients

The compositions herein can contain a variety of other optional components suitable for rendering such compositions more cosmetically or aesthetically acceptable or to provide them with additional usage benefits. Such conventional optional ingredients are well-known to those skilled in the art. These include any cosmetically acceptable ingredients such as those found in the *CTFA International Cosmetic Ingredient Dictionary and Handbook, 7th edition, edited by Wenninger and McEwen, (The Cosmetic, Toiletry, and Fragrance Association, Inc., Washington, D.C., 1997*). As used herein "cosmetically acceptable" means a material (e.g., compound or composition) which is suitable for use in contact with skin, hair or other suitable substrate as defined hereinbelow.

### Nonvolatile solvents

While the compositions of the present invention must comprise a volatile solvent they may also comprise "nonvolatile" solvents. Suitable non-volatile solvents include, but are not limited to, benzyl benzoate, diethyl phthalate, isopropyl myristate, and mixtures thereof.

### Molecular wedges

Particularly preferred for use herein for providing increased longevity and strength of odour of fragrance is a low molecular weight polyol molecular wedge having from about 2 to about 12 carbon atoms, preferably from about 2 to about 6 carbon atoms and at least one -OH functional group, preferably at least 2 -OH functional groups. These polyols can further contain ether groups within the carbon chain. Suitable examples include ethylene glycol, propylene glycol, dipropylene glycol, 1,4-butanediol, 1,6-hexanediol and mixtures thereof. When present these polyols are present at a level of from about 0.01% to about 20%, preferably from about 0.1% to about 10%, and especially from about 0.5% to about 5% by weight of composition. It is preferred that the molar ratio of molecular wedge material to oligosaccharide is from 10:1 to 1:10, preferably 1:1 or greater, especially 1:1.

While not wishing to be limited by theory, the above mentioned molecular wedge molecules form tertiary inclusion complexes with the complexed perfume material and the cyclic oligosaccharide. These small dipolar molcules can fit into the cavity of the cyclic oligosaccharide and anchor via their OH groups onto the outside rim of the cyclic oligosaccharide through hydrogen bonding. This enables the inclusion of all or parts of the fragrance material into the cavity of the cyclic oligosaccharide such that the stability of the formed tertiary complex is increased versus the complex formed by the fragrance material and cyclic oligosaccharide alone.

### Water

The compositions of the present invention may also comprise water. If present, the water will preferably comprise from about 0.1% to about 40%, more preferably from about 1% to about 30%, even more preferably about 5% to about 20%, by weight, of total composition.

There are a number of other examples of additional ingredients that are suitable for inclusion into the present compositions. These include, but are not limited to, alcohol denaturants such as denatonium benzoate; UV stabilisers such as benzophenone-2; antioxidants such as tocopheryl acetate; preservatives such as phenoxyethanol, benzyl alcohol, methyl paraben, propyl paraben; dyes; pH adjusting agents such as lactic acid, citric acid, sodium citrate, succinic acid, phosphoric acid, sodium hydroxide, sodium carbonate; deodorants and antimicrobials such as famesol and zinc phenolsulphonate; humectants such as glycerine; oils; skin conditioning agents such as allantoin; cooling agents such as trimethyl isopropyl butanamide and menthol; hair conditioning ingredients such as panthenol, panthetine, pantotheine, panthenyl ethyl ether, and combinations thereof; silicones; solvents such as hexylene glycol; hair-hold polymers such as those described in WO-A-94/08557; salts in general, such as potassium acetate and sodium chloride and mixtures thereof. If present, these additional ingredients will preferably be present at a level of less than 10%, by weight, of total composition. More preferably these additional ingredients will be present at a level of less than 5%, by weight, of total composition.

### Methods of Use

The compositions of the present invention can be used to provide a long-lasting fragrance to a suitable substrate. As used herein the term "suitable substrate" means any surface to which the present composition may be applied without an unduly adverse effect. Suitable substrates include, but are not limited to, skin, hair and fabrics. Preferably the present compositions are applied to skin or hair, especially skin.

The cosmetic compositions of the present invention may be used in a conventional manner for fragrancing a suitable substrate. An effective amount of the composition, typically from about 1µl to about 1000µl, preferably from about 10µl to about 250µl, more preferably from about 25µl to about 100µl, is applied to the substrate. The composition may be applied by hand but is preferably applied utilising a vaporiser. Preferably, the composition is then left to dry.

Therefore, the preferred method of treating the substrate comprises:
(a) applying an effective amount of the composition to a suitable substrate; and preferably
(b) allowing the composition to dry.

### Product Forms

The compositions of the present invention may take any form suitable for cosmetic use. These include, but are not limited to, vapour sprays, aerosols, emulsions, lotions and liquids. Preferably the compositions of the present invention take the form of a vapour spray.

### Examples

The following examples further illustrate the preferred embodiments within the scope of the present invention. The examples are given solely for the purposes of illustration and are not to be construed as limitations of the present invention as many variations of the invention are possible without departing from its scope. Unless otherwise indicated, all ingredients are expressed on a weight percentage of the active ingredient.

| | I (% wt) Vapour Spray | II (% wt) Vapour Spray | III (% wt) Vapour Spray |
|---|---|---|---|
| Fragrance | 15 | 12.5 | 10 |
| Cyclic oligosaccharide¹ | 5 | 7.5 | 10 |
| Ethanol | 70 | 70 | 70 |
| Deionised Water | 10 | 10 | 10 |

| | | | |
|---|---|---|---|
| 1) Beta W7 M1.8 available from Wacker-Chemie GmbH, Hanns-Seidel-Platz 4, Munchen, DE | | | |

The cyclic oligosaccharide was dissolved in the ethanol at room temperature, with stirring. Then the fragrance and water were added with stirring.

| | IV (% wt) Liquid Perfume | V (% wt) Liquid Perfume | VI (% wt) Liquid Perfume |
|---|---|---|---|
| Fragrance | 15 | 12.5 | 10 |
| Cyclic oligosaccharide¹ | 5 | 7.5 | 10 |
| Ethanol | 80 | 80 | 80 |

| | | | |
|---|---|---|---|
| 1) Beta W7 M1.8 available from Wacker-Chemie GmbH, Hanns-Seidel-Platz 4, Munchen, DE | | | |

The cyclic oligosaccharide was dissolved in the ethanol at room temperature, with stirring. Then the fragrance was added with stirring.

| | VII (% wt) Deodorant | VIII (% wt) Deodorant | IX (% wt) Deodorant |
|---|---|---|---|
| Fragrance | 3 | 2.5 | 3 |
| Cyclic oligosaccharide¹ | 1.5 | 2 | 1.5 |
| Zinc phenolsulphonate | 2 | 1 | 2 |
| Dipropylene Glycol | 31 | 17.5 | 27 |
| Isopropyl myristate | 1.5 | 7 | 1.5 |
| Ethanol | 61 | 70 | 65 |

| | | | |
|---|---|---|---|
| 1) Beta W7 M1.8 available from Wacker-Chemie GmbH, Hanns-Seidel-Platz 4, Munchen, DE | | | |

The zinc phenolsulphonate is stirred into the ethanol until fully dissolved. Then the dipropylene glycol is added with stirring. Next the isopropyl myristate, then the cyclic oligosaccharide and then the fragrance are all added with stirring. All steps are performed at room temperature. For an aerosol deodorant a propellant such as propane butane (CAP 40®) can be added to Examples VII-IX according to standard industry practice.

### Examples X-XI

| | X (% Wt) Vapour Spray | XI (% wt) Vapour Spray |
|---|---|---|
| Fragrance | 15 | 15 |
| Cyclic oligosaccharide¹ | 5 | 5 |
| Ethanol | 70 | 70 |
| Deionised Water | to 100 | to 100 |
| dipropylene glycol | 5 | 2.5 |
| ethylene glycol | 0 | 2.5 |

| | | |
|---|---|---|
| 1) Beta W7 M1.8 available from Wacker-Chemie GmbH, Hanns-Seidel-Platz 4, Munchen, DE | | |

The cyclic oligosaccharide was dissolved in the ethanol at room temperature, with stirring. Then the fragrance, dipropylene glycol/ethylene glycol and water were added with stirring.

All of the above examples were found to give an initial 'burst' of fragrance and have long-lasting fragrance.

## Claims

1. A cosmetic composition comprising:
(a) fragrance;
(b) cyclic oligosaccharide having one or more unsubstituted alkyl substituents; and
(c) 50% or greater, by weight, of volatile solvent.

2. A composition according to Claim 1 wherein said cyclic oligosaccharide has six, seven or eight saccharide units and mixtures thereof, preferably seven saccharide units.

3. A composition according to any of the preceding claims wherein said cyclic oligosaccharide is a cyclodextrin.

4. A composition according to any of the preceding claims wherein said alkyl substituent is selected from C₁-C₈ alkyl groups and mixtures thereof, preferably C₁-C₄ alkyl groups and mixtures thereof.

5. A composition according to any of the preceding claims wherein said alkyl substituent is a methyl group.

6. A composition according to any of the preceding claims wherein said solvent is selected from C₁-C₄ alcohols and mixtures thereof.

7. A composition according to any of the preceding claims wherein said solvent is ethanol.

8. A composition according to any of the preceding claims comprising 55% or greater, preferably 60% or greater, by weight, of said solvent.

9. A composition according to any of the preceding claims wherein said fragrance comprises from about 0.01% to about 40%, preferably from about 2.5% to about 25%, by weight, of total composition.

10. A composition according to any of the preceding claims wherein said cyclic oligosaccharide comprises from about 0.001% to about 40%, preferably from about 1% to about 20%, by weight, of total composition.

11. A composition according to any of the preceding claims comprising from about 0.1% to about 40%, preferably from about 1% to about 30%, more preferably about 5% to about 20%, by weight, water.

12. A composition according to any of the preceding claims comprising a polyol having from about 2 to about 12 carbon atoms, preferably from about 2 to about 6 carbon atoms, and at least one -OH group, preferably at least two -OH groups, more preferably two terminal -OH groups.

13. A composition according to Claim 12 wherein the polyol is selected from ethylene glycol, propylene glycol, dipropylene glycol, 1,4 butanediol and 1,6 hexanediol, and mixtures thereof.

14. A cosmetic method of fragrancing a substrate comprising:
(a) applying a composition according to any of Claims 1-13 to said substrate; and preferably
(b) allowing said composition to dry.

15. Use of a composition according to any of Claims 1-13 for providing a long-lasting fragrance to skin or hair.

## Patentansprüche

1. Kosmetische Zusammensetzung, umfassend:
(a) Riechstoff;
(b) cyclisches Oligosaccharid mit einem oder mehreren unsubstituierten Alkylsubstituenten; und
(c) 50 Gew.-% oder mehr flüchtiges Lösungsmittel.

2. Zusammensetzung nach Anspruch 1, wobei das cyclische Oligosaccharid sechs, sieben oder acht Saccharideinheiten und Mischungen hiervon aufweist, vorzugsweise sieben Saccharideinheiten.

3. Zusammensetzung nach mindestens einem der vorangehenden Ansprüche, wobei das cyclische Oligosaccharid ein Cyclodextrin ist.

4. Zusammensetzung nach mindestens einem der vorangehenden Ansprüche, wobei der Alkylsubstituent gewählt ist aus C₁-C₈-Alkylgruppen und Mischungen hiervon, vorzugsweise C₁-C₄-Alkylgruppen und Mischungen hiervon.

5. Zusammensetzung nach mindestens einem der vorangehenden Ansprüche, wobei der Alkylsubstituent eine Methylgruppe ist.

6. Zusammensetzung nach mindestens einem der vorangehenden Ansprüche, wobei das Lösungsmittel aus C₁-C₄-Alkoholen und Mischungen hiervon gewählt ist.

7. Zusammensetzung nach mindestens einem der vorangehenden Ansprüche, wobei das Lösungsmittel Ethanol ist.

8. Zusammensetzung nach mindestens einem der vorangehenden Ansprüche, umfassend 55 Gew.-% oder mehr, vorzugsweise 60 Gew.-% oder mehr des Lösungsmittels.

9. Zusammensetzung nach mindestens einem der vorangehenden Ansprüche, wobei der Riechstoff etwa 0,01 bis etwa 40 Gew.-%, vorzugsweise etwa 2,5 bis etwa 25 Gew.-% der Gesamtzusammensetzung umfasst.

10. Zusammensetzung nach mindestens einem der vorangehenden Ansprüche, wobei das cyclische Oligosaccharid etwa 0,001 bis etwa 40 Gew.-%, vorzugsweise etwa 1 bis etwa 20 Gew.-% der Gesamtzusammensetzung umfasst.

11. Zusammensetzung nach mindestens einem der vorangehenden Ansprüche, umfassend etwa 0,1 bis etwa 40 Gew.-%, vorzugsweise etwa 1 bis etwa 30 Gew.-%, weiter vorzugsweise etwa 5 bis etwa 20 Gew.-% Wasser.

12. Zusammensetzung nach mindestens einem der vorangehenden Ansprüche, umfassend ein Polyol mit etwa 2 bis etwa 12 Kohlenstoffatomen, vorzugsweise etwa 2 bis etwa 6 Kohlenstoffatomen, und mindestens einer -OH-Gruppe, vorzugsweise mindestens zwei -OH-Gruppen, weiter vorzugsweise zwei endständigen -OH-Gruppen.

13. Zusammensetzung nach Anspruch 12, wobei das Polyol gewählt ist aus Ethylenglykol, Propylenglykol, Dipropylenglykol, 1,4-Butandiol und 1,6-Hexandiol und Mischungen hiervon.

14. Kosmetisches Verfahren zur Aromatisierung eines Susbtrats, umfassend:
(a) Aufbringen einer Zusammensetzung gemäß mindestens einem der Ansprüche 1-13 auf das Substrat; und vorzugsweise
(b) Trocknenlassen der Zusammensetzung.

15. Verwendung einer Zusammensetzung nach mindestens einem der Ansprüche 1-13, um die Haut oder das Haar mit einem lang anhaltenden Riechstoff zu versehen.

## Revendications

1. Composition cosmétique comprenant:
(a) un parfum ;
(b) un oligosaccharide cyclique ayant un ou plusieurs substituants alkyles non substitués ; et
(c) 50 % ou plus, en poids, de solvant volatil.

2. Composition selon la revendication 1, dans laquelle ledit oligosaccharide cyclique a six, sept ou huit motifs saccharides et leurs mélanges, de préférence sept motifs saccharides.

3. Composition selon l'une quelconque des revendications précédentes, dans laquelle ledit oligosaccharide cyclique est une cyclodextrine.

4. Composition selon l'une quelconque des revendications précédentes, dans laquelle ledit substituant alkyle est choisi parmi les groupes alkyles en C₁ à C₈ et leurs mélanges, de préférence des groupes alkyles en C₁ à C₄ et leurs mélanges.

5. Composition selon l'une quelconque des revendications précédentes, dans laquelle ledit substituant alkyle est un groupe méthyle.

6. Composition selon l'une quelconque des revendications précédentes, dans laquelle ledit solvant est choisi parmi les alcools en C₁ à C₄ et leurs mélanges.

7. Composition selon l'une quelconque des revendications précédentes, dans laquelle ledit solvant est l'éthanol.

8. Composition selon l'une quelconque des revendications précédentes, comprenant 55 % ou plus, de préférence 60 % ou plus, en poids, dudit solvant.

9. Composition selon l'une quelconque des revendications précédentes, dans laquelle ledit parfum représente d'environ 0,01 % à environ 40 %, de préférence d'environ 2,5 % à environ 25 %, en poids, de la composition totale.

10. Composition selon l'une quelconque des revendications précédentes, dans laquelle ledit oligosaccharide cyclique représente d'environ 0,001 % à environ 40 %, de préférence d'environ 1 % à environ 20 %, en poids, de la composition totale.

11. Composition selon l'une quelconque des revendications précédentes, comprenant d'environ 0,1 % à environ 40 %, de préférence d'environ 1 % à environ 30 %, plus préférablement encore d'environ 5 % à environ 20 %, en poids, d'eau.

12. Composition selon l'une quelconque des revendications précédentes, comprenant un polyol ayant d'environ 2 à environ 12 atomes de carbone, de préférence d'environ 2 à environ 6 atomes de carbone, et au moins un groupe -OH, de préférence au moins deux groupes -OH, plus préférablement encore deux groupes -OH terminaux.

13. Composition selon la revendication 12, dans laquelle le polyol est choisi parmi l'éthylèneglycol, le propylèneglycol, le dipropylèneglycol, le 1,4 butanediol et le 1,6 hexanediol, et leurs mélanges.

14. Procédé cosmétique pour parfumer un substrat, comprenant les étapes consistant à :
(a) appliquer une composition selon l'une quelconque des revendications 1 à 13 audit substrat ; et de préférence
(b) laisser ladite composition sécher.

15. Utilisation d'une composition selon l'une quelconque des revendications 1 à 13 pour fournir un parfum de longue durée à la peau ou aux cheveux.
